(19) Europäisches Patentamt
European Patent Office
Office européen des brevets

(11) **EP 4 785 862 A1**

(12) **EUROPEAN PATENT APPLICATION**
published in accordance with Art. 153(4) EPC

(43) Date of publication:
**05.08.2026 Bulletin 2026/32**

(21) Application number: **23953884.6**

(22) Date of filing: **17.10.2023**

(51) International Patent Classification (IPC):
***A61B 5/055*** *(2006.01)*

(86) International application number:
**PCT/CN2023/125056**

(87) International publication number:
**WO 2025/065748 (03.04.2025 Gazette 2025/14)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **27.09.2023 CN 202311266353**

(71) Applicant: **Tsinghua University
Beijing 100084 (CN)**

(72) Inventors:
• **SONG, Xiaolei
Beijing 100084 (CN)**
• **LIU, Chuyu
Beijing 100084 (CN)**
• **GAO, Nan
Beijing 100084 (CN)**

(74) Representative: **M. Zardi & Co S.A.
Via G. B. Pioda, 6
6900 Lugano (CH)**

(54) **VARIABLE SATURATION GRADIENT-BASED THREE-DIMENSIONAL CEST OR Z-SPECTRUM MAGNETIC RESONANCE IMAGING METHOD AND DEVICE**

(57) Provided are a variable saturation gradient-based three-dimensional Chemical Exchange Saturation Transfer (CEST) or Z-spectrum magnetic resonance imaging method and an electronic device. The method includes: applying a saturation pulse to a target object while turning on a gradient in an *r*-direction, reading out a raw image, and repeating the above process *n* times to obtain *n* sets of raw images; filling the *n* sets of raw images into an *r*-$\Delta\omega$ plane; determining a target uncollected image based on a collected image in the *r*-$\Delta\omega$ plane, and estimating the target uncollected image; and performing interpolation and/or fitting based on the collected image and the estimated target uncollected image to obtain a CEST or Z-spectrum image having a high spatial resolution and a high spectral sampling rate.

Applying a saturation pulse to a target object while turning on a gradient in an *r*-direction, reading out a raw image, and repeating the above process *n* times to obtain *n* sets of raw images, in which a center frequency of the saturation pulse applied each time and a value of the gradient are different, *r* represents a spatial position and includes at least one of *x*-direction, *y*-direction, or *z*-direction, and *n* is an integer greater than 1 — S1

Filling the *n* sets of raw images into an *r*-$\Delta\omega$ plane, where $\Delta\omega$ represents a frequency offset — S2

Determining a target uncollected image based on a collected image in the *r*-$\Delta\omega$ plane, and estimating the target uncollected image — S3

Performing interpolation and/or fitting based on the collected image and the estimated target uncollected image to obtain a CEST or Z-spectrum image having a high spatial resolution and a high spectral sampling rate — S4

FIG. 1

EP 4 785 862 A1

## Description

## CROSS-REFERENCE TO RELATED APPLICATION

This application claims priority to Chinese Patent Application No. 202311266353.1, filed on September 27, 2023, and titled "VARIABLE SATURATION GRADIENT-BASED THREE-DIMENSIONAL CEST OR Z-SPECTRUM MAGNETIC RESONANCE IMAGING METHOD AND DEVICE", the entire contents of which are incorporated herein by reference.

## FIELD

The present disclosure relates to the field of magnetic resonance imaging technologies, and more particularly, to a variable saturation gradient-based three-dimensional Chemical Exchange Saturation Transfer (CEST) magnetic resonance imaging method and an electronic device.

## BACKGROUND

CEST Magnetic Resonance Imaging (MRI) is an emerging molecular imaging technology that can be used for metabolic detection and pH detection in diseases such as cancer, tumors, and stroke.

A frequency of hydrogen protons in substances such as proteins, glucose, creatine, and salicylic acid analogs in the human body differs from a resonance frequency of hydrogen protons in water molecules. In addition, magnetization vectors of the hydrogen protons in these substances can undergo an exchange effect with magnetization vectors of the hydrogen protons in water. In view of this, saturation radio frequency pulses at a specific frequency can be applied to human tissues to saturate the hydrogen protons in these chemical substances, which in turn affects a water signal intensity through the exchange effect. By detecting changes in the water signal intensity, information regarding exchange rates and concentrations of various chemical substances within the human body can be indirectly reflected. Therefore, CEST technology can serve as a signal amplifier for low-concentration biomolecules. Typically, CEST requires collecting MR images under saturation pulses at different frequencies, and then plotting a Z-spectrum to study chemical exchange information at a specific frequency offset. By analyzing the Z-spectrum, a desired CEST signal can be extracted.

However, since CEST requires sequentially collecting images of a same anatomical structure at a plurality of saturation frequencies, and saturation pulses lasting several seconds need to be repeatedly applied, CEST MRI technology faces a problem of a long scan duration. To keep the scan duration within a clinically acceptable range, conventional CEST sequences usually compromise on a quantity of saturation frequency points and a spatial resolution. How to achieve fast imaging with both high spatial resolution and high spectral sampling rate has been a research hotspot in the field of CEST MRI.

In recent years, a series of technologies have emerged to solve the problems of the long scan duration and limited spatial-spectral sampling rates in CEST MRI. In terms of collection sequences, employing a fast readout module or a steady-state CEST sequence enables whole-brain scanning. However, since each saturation frequency offset requires repeating at least one Repetition Time (TR) duration, and each TR duration requires four seconds to six seconds to ensure a sufficient saturation duration and longitudinal relaxation recovery, it is difficult in practice to collect CEST data with both the spatial resolution and the spectral sampling rate. To further improve a collection efficiency, a saturation gradient encoding technique is provided in the related art. This technique achieves a simultaneous collection of a plurality of saturation frequencies by turning on a gradient in a slice-selection direction during application of saturation pulses, resulting in an intra-slice encoded ultrafast Z-spectrum imaging sequence. However, since this technique performs Z-spectrum reading in the slice-selection direction, i.e., a slice having a thickness of 10 mm is divided into dozens to hundreds of sub-slices encoded by different saturation frequency offsets, only tissues that are relatively uniform across these sub-slices can be imaged. Further, due to an excessively thin thickness ($100 \, \mu m$) of each sub-slice, the sub-slice is limited by a signal-to-noise ratio, and thus only a low intra-slice resolution (5 mm $\times$ 5 mm to 8 mm) can be achieved, which restricts a further application of this technique. In terms of reconstruction, some techniques use methods such as parallel imaging, compressed sensing, and deep learning to recover a complete CEST image from undersampled data, accelerating CEST imaging. However, these techniques have a disadvantage that reconstruction after undersampling the data usually trades off an image quality, which is unfavorable for a subsequent quantitative CEST analysis.

## SUMMARY

The present disclosure aims to solve at least one of the technical problems in the related art to some extent. To this end, an objective of the present disclosure is to provide a variable saturation gradient-based three-dimensional CEST or Z-spectrum magnetic resonance imaging method and an electronic device, to rapidly obtain a three-dimensional CEST or Z-spectrum magnetic resonance image having a high spatial resolution and a high spectral sampling rate.

To solve the above technical problems, according to an embodiment in a first aspect of the present disclosure, a variable saturation gradient-based three-dimensional CEST or Z-spectrum magnetic resonance imaging method is provided. The method includes: applying a saturation pulse to a target object while turning on a gradient in an *r*-direction, reading out a raw image, and repeating the above process *n* times to obtain *n* sets of raw images, in which a center frequency of the saturation pulse applied and a value of the gradient are different for each time, *r* represents a spatial position and includes at least one of *x*-direction, *y*-direction, or *z*-direction, and *n* is an integer greater than 1; filling the *n* sets of raw images into an *r*-$\Delta\omega$ plane, where $\Delta\omega$ represents a frequency offset; determining a target uncollected image based on a collected image in the *r*-$\Delta\omega$ plane, and estimating the target uncollected image; and performing interpolation and/or fitting based on the collected image and the estimated target uncollected image to obtain a CEST or Z-spectrum image having a high spatial resolution and a high spectral sampling rate.

With the variable saturation gradient-based three-dimensional CEST or Z-spectrum magnetic resonance imaging method according to the embodiments of the present disclosure, the saturation pulse to the target object is applied while turning on the gradient in the *r*-direction. A raw image is read out. The above process is repeated *n* times to obtain the *n* sets of raw images. The *n* sets of raw images are filled into the *r*-$\Delta\omega$ plane. The target uncollected image is determined based on the collected image in the *r*-$\Delta\omega$ plane and the target uncollected image is estimated. The interpolation and/or the fitting is performed based on the collected image and the estimated target uncollected image to obtain a CEST-Z-spectrum image having a high spatial resolution and a high spectral sampling rate. With the method, a CEST magnetic resonance image having a high spatial resolution, a high spectral sampling rate, and strong interpretability can be rapidly obtained, which helps improve accuracy of a CEST quantitative analysis and a disease diagnosis, has important clinical value, and offers high flexibility and satisfactory scalability.

In addition, the variable saturation gradient-based three-dimensional CEST or Z-spectrum magnetic resonance imaging method according to the embodiments of the present disclosure may further have the following additional technical features.

According to an embodiment of the present disclosure, the *n* sets of raw images are filled into the *r*-$\Delta\omega$ plane using the following equation: **[0012]**

$$\Delta\omega(r) = \Delta\omega_0 + \gamma G_{\text{offset}} \, \Delta r \,,$$

where $\Delta\omega(r)$ represents a frequency offset corresponding to the *r*-direction, $\Delta r$ represents a distance between an imaging position corresponding to the *r*-direction and a magnetic field zero point, $\gamma$ represents a gyromagnetic ratio, $\Delta\omega_0$ represents a frequency offset between a saturation pulse frequency and a water proton, and $G_{\text{offset}}$ represents the gradient in the *r*-direction that is turned on during application of the saturation pulse.

According to an embodiment of the present disclosure, the target uncollected image includes an uncollected image, in the *r*-$\Delta\omega$ plane, on an adjacent slice and at an adjacent saturation frequency offset relative to the collected image.

According to an embodiment of the present disclosure, the collected images are denoted as $I_{r_1, \omega_1}$ and $I_{r_2, \omega_2}$. The target uncollected images corresponding to the collected images include $I_{r_1, \omega_2}$ and $I_{r_2, \omega_1}$, and and $I_{r_2, \omega_1}$ are estimated through the following equations, respectively:

$$I_{r_1, \omega_2} = I_{r_1, \omega_1} \cdot \frac{\overline{I_{r_2, \omega_2}}}{\overline{I_{r_1, \omega_1}}} * \frac{\overline{M_{r_1}}}{\overline{M_{r_2}}}$$

$$I_{r_2, \omega_1} = I_{r_2, \omega_2} \cdot \frac{\overline{I_{r_1, \omega_1}}}{\overline{I_{r_2, \omega_2}}} * \frac{\overline{M_{r_2}}}{\overline{M_{r_1}}},$$

where $M_{r_1}$ and $M_{r_2}$ represent unsaturated images on slice *r*1 and slice *r*2, respectively; $I_{zr_1, \omega_1}$ and $I_{zr_2, \omega_2}$ represent a collected image at frequency offset $\omega_1$ on the slice *r*1 and a collected image at frequency offset $\omega_2$ on the slice *r*2, respectively; $I_{r_1, 2}$ and $I_{r_2, \omega_1}$ represent a target uncollected image at the frequency offset $\omega_2$ on the slice *r*1 and a target uncollected image at the frequency offset $\omega_1$ on the slice *r*2, respectively; $S_{r_1, \omega_2}$ and $S_{r_2, \omega_1}$ represent relative saturation information at the frequency offset $\omega_2$ on the slice *r*1 and relative saturation information at the frequency offset $\omega_1$ on the slice *r*2, respectively; $\overline{I_{r_2, \omega_2}}$ and $\overline{I_{r_1, \omega_1}}$ represent an average value of the collected image at the frequency offset $\omega_1$ on the slice *r*1 and an average value of the collected image at the frequency offset $\omega_2$ on the slice *r*2, respectively; and $\overline{M_{r_1}}$ and $\overline{M_{r_2}}$ represent average values of the unsaturated images on the slice *r*1 and the slice *r*2, respectively.

According to an embodiment of the present disclosure, the performing the interpolation and/or the fitting based on the collected image and the estimated target uncollected image includes: performing simulation using a multi-pool Bloch-McConnell equation, and obtaining a Z-spectrum sample set with a plurality of different waveforms by varying a simulation parameter of each pool; performing singular value decomposition on the Z-spectrum sample set, and selecting right singular vectors corresponding to $m$ largest singular values to form a new singular vector matrix, where $m$ is an integer greater than 2; and determining all vectors z0 having incomplete saturation frequency offset points based on the collected image and the estimated target uncollected image, and obtaining, for each of all the vectors z0, a complete spectrum of a pixel point corresponding to the vector z0 based on the vector z0 and the new singular vector matrix.

According to an embodiment of the present disclosure, a value of the gradient includes at least 0.

According to an embodiment of the present disclosure, the $n$ sets of raw images corresponding to $n$ slices are collected using a fast spin echo method, a gradient echo method, or an echo planar method.

According to an embodiment of the present disclosure, the $n$ sets of raw images collected corresponding to $n$ slices are arranged in a radial pattern, a parallel pattern, or an intersecting pattern on the $r$-$\Delta\omega$ plane.

According to an embodiment of the present disclosure, the variable saturation gradient-based three-dimensional CEST or Z-spectrum magnetic resonance imaging method further includes: performing a post-processing on the obtained CEST-Z-spectrum image having the high spatial resolution and the high spectral sampling rate. The post-processing includes a magnetization transfer ratio asymmetry processing, Lorentzian difference, and a multi-pool Lorentzian processing.

To solve the above technical problems, according to an embodiment in a second aspect of the present disclosure, an electronic device is provided. The electronic device includes a memory, a processor, and a computer program stored on the memory. The computer program, when executed by the processor, implements the variable saturation gradient-based three-dimensional CEST or Z-spectrum magnetic resonance imaging method according to any of the above embodiments.

With the electronic device according to the embodiments of the present disclosure, the computer program stored on the memory and corresponding to the variable saturation gradient-based three-dimensional CEST or Z-spectrum magnetic resonance imaging method according to the above embodiments is executed by the processor. Thus, the CEST magnetic resonance image having the high spatial resolution, the high spectral sampling rate, and the strong interpretability can be rapidly obtained, which helps improve the accuracy of the CEST quantitative analysis and the disease diagnosis, and has the important clinical value.

Additional aspects and advantages of the present disclosure will be provided at least in part in the following description, or will become apparent at least in part from the following description, or can be learned from practicing of the present disclosure.

## BRIEF DESCRIPTION OF THE DRAWINGS

FIG. 1 is a flowchart of a variable saturation gradient-based three-dimensional CEST or Z-spectrum magnetic resonance imaging method according to an embodiment of the present disclosure.

FIG. 2(a) is a schematic diagram of a collection sequence of a collection scheme according to an example of the present disclosure.

FIG. 2(b) is a raw image collected using the collection scheme illustrated in FIG. 2(a).

FIG. 2(c) is a schematic diagram showing the raw image illustrated in FIG. 2(b) filled into an $r$-$\Delta\omega$ plane.

FIG. 2(d) is a schematic diagram of frequency offset points of the raw image illustrated in FIG. 2(b).

FIG. 3 is a schematic diagram showing a raw image according to some examples of the present disclosure filled into an $r$-$\Delta\omega$ plane.

FIG. 4 is a schematic diagram of a nearest-neighbor estimation process according to an embodiment of the present disclosure.

FIG. 5 is a schematic diagram of a fitting estimation process according to an embodiment of the present disclosure.

FIG. 6 is an effect diagram obtained after a post-processing according to an embodiment of the present disclosure.

FIG. 7 is a structural block diagram of an electronic device according to an embodiment of the present disclosure.

## DETAILED DESCRIPTION

Embodiments of the present disclosure will be described in detail below with reference to examples thereof as illustrated in the accompanying drawings, throughout which same or similar elements, or elements having same or similar functions, are denoted by same or similar reference numerals. The embodiments described below with reference to the drawings are illustrative only, and are intended to explain, rather than limit, the present disclosure.

A variable saturation gradient-based three-dimensional CEST or Z-spectrum magnetic resonance imaging method and an electronic device according to embodiments of the present disclosure are described below with reference to the accompanying drawings.

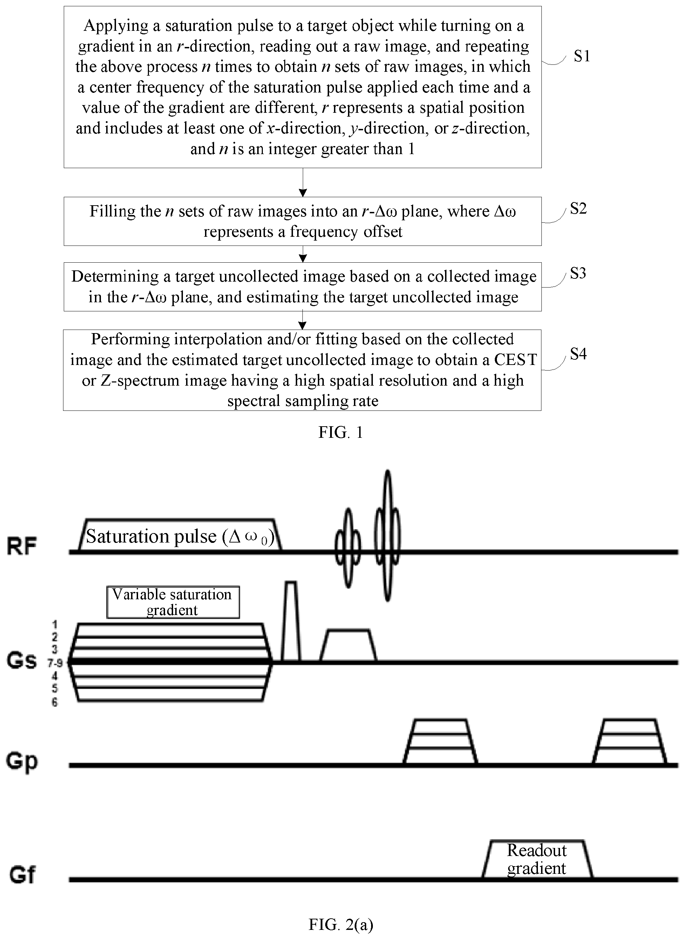

FIG. 1 is a flowchart of a variable saturation gradient-based three-dimensional CEST or Z-spectrum magnetic resonance imaging method according to an embodiment of the present disclosure.

As illustrated in FIG. 1, the variable saturation gradient-based three-dimensional CEST or Z-spectrum magnetic resonance imaging method includes operations at blocks.

At S1, a saturation pulse is applied to a target object while turning on a gradient in an $r$-direction, a raw image is read out, and the above process is repeated $n$ times to obtain $n$ sets of raw images. A center frequency of the saturation pulse applied and a value of the gradient are different for each time. $r$ represents a spatial position and includes at least one of $x$-direction, $y$-direction, or $z$-direction. $n$ is an integer greater than 1.

In an embodiment of the present disclosure, the target object may be any object capable of being imaged through magnetic resonance CEST, such as the brain, abdominal organs, thoracic organs, muscles, or the like of a patient.

In some examples of the present disclosure, a value of $n$ is less than 10, a frequency of the saturation pulse ranges from -15 ppm to 15 ppm, and a value of the gradient includes at least 0, for example, ranging from -1 $G_{offset}$ to 1 $G_{offset}$.

FIG. 2(a) illustrates a scheme for a high-spatial and high-resolution CEST-MRI image collection based on spatial-frequency encoding. In this scheme, n=9. That is, the value $G_{offset}$ of the gradient changes nine times, and a saturation pulse frequency ppm also changes nine times accordingly. Specific values are: 1: 1 $G_{offset}$+0 ppm, 2: 0.5 $G_{offset}$+0 ppm, 3: 0.25 $G_{offset}$+0 ppm, 4: -1 $G_{offset}$+0 ppm, 5: -0.5 $G_{offset}$+0 ppm, 6: -0.25 $G_{offset}$+0 ppm, 7: 0 $G_{offset}$+(-15 ppm), 8: 0 $G_{offset}$+3.5 ppm, 9: 0 $G_{offset}$+(-3.5 ppm).

It should be noted that, in FIG. 2(a), RF represents a radio frequency pulse, Gs represents a direction selection gradient, Gp represents a phase encoding gradient, and Gf represents a frequency encoding gradient/readout gradient. For each time, the saturation pulse applied and the turned-on gradient in the $r$-direction may be any designed values.

At S2, the $n$ sets of raw images are filled into an $r$-$\Delta\omega$ plane, where $\Delta\omega$ represents a frequency offset.

Specifically, as illustrated in FIG. 2(a), a collection sequence is characterized in that, a CEST saturation frequency is applied while turning on a variable gradient in a slice selection direction. Thus, hydrogen protons in different anatomical slices in the $r$-direction can precess at different frequencies, which in turn generates, relative to an intrinsic frequency of the saturation pulse, a frequency offset that varies with a slice position. A relationship between the frequency offset and the slice position is:

$$\Delta\omega(r) = \Delta\omega_0 + \gamma G_{offset}\, \Delta r \,,$$

where $\Delta\omega(r)$ represents a frequency offset corresponding to the $r$-direction, $\Delta r$ represents a distance between an imaging position corresponding to the $r$-direction and a magnetic field zero point, $\gamma$ represents a gyromagnetic ratio, $\Delta\omega_0$ represents a frequency offset between a saturation pulse frequency and a water proton, and $G_{offset}$ represents the gradient in the $r$-direction that is turned on during application of the saturation pulse.

After the above spatial-frequency encoding process, an actual saturation frequency in different spatial directions varies linearly with the spatial position in the slice selection direction. The collected CEST MRI data contains entire spatial positions of the target object and a plurality of saturation frequency offsets. By adjusting a magnitude of the gradient $G_{offset}$ and the intrinsic frequency of the saturation pulse, any straight line on the $r$-$\Delta\omega$ plane can be collected. Thus, full spatial-spectral high-resolution CEST data, which would otherwise require at least 200 collections, can be obtained in fewer than ten (e.g., nine) collections, improving a data collection efficiency.

In this embodiment, the $n$ sets of raw images corresponding to $n$ slices may be collected using, but not limited to, a fast spin echo method, a gradient echo method, or an echo planar method.

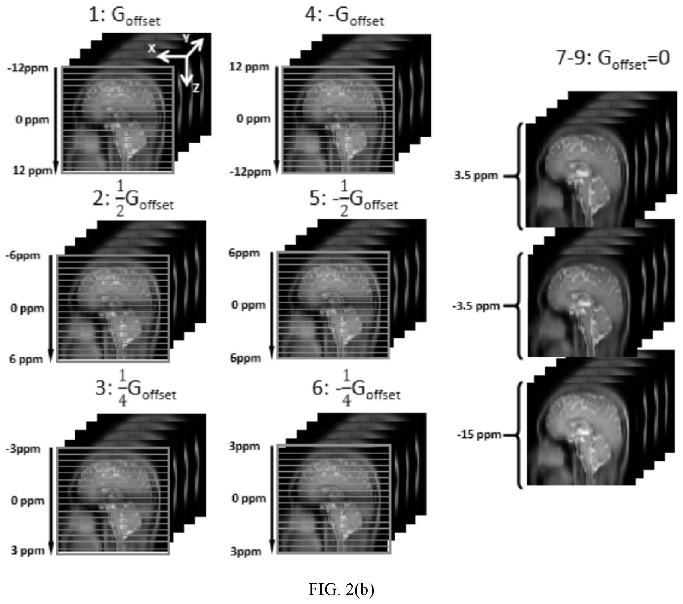

FIG. 2(b) illustrates a raw image collected using the collection scheme illustrated in FIG. 2(a), containing nine sets of whole-brain high-spatial-resolution CEST MRI images.

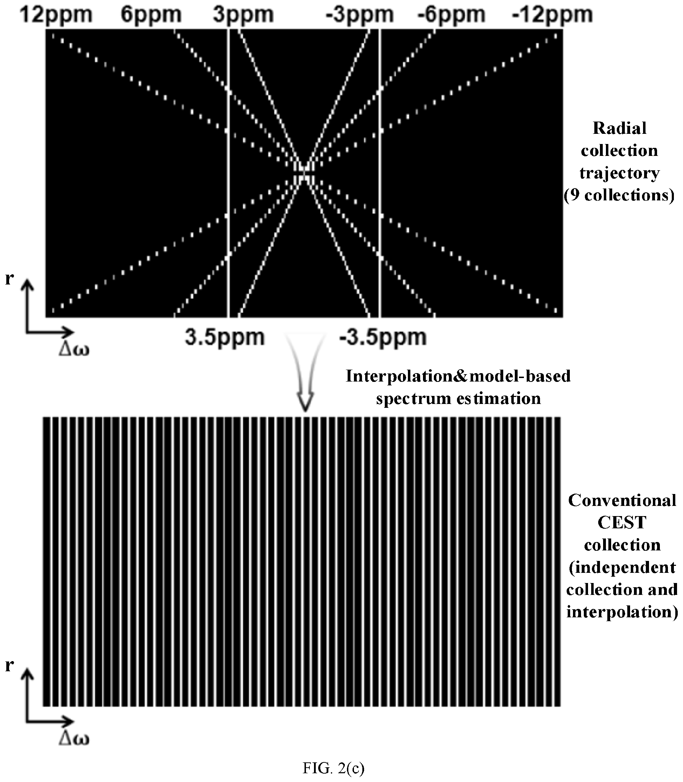

FIG. 2(c) illustrates a schematic diagram of filling the collected whole-brain high-resolution raw images illustrated in FIG. 2(b) into the $r$-$\Delta\omega$ plane. For the collection scheme illustrated in FIG. 2(a), the collected raw images are arranged in a radial pattern on the $r$-$\Delta\omega$ plane.

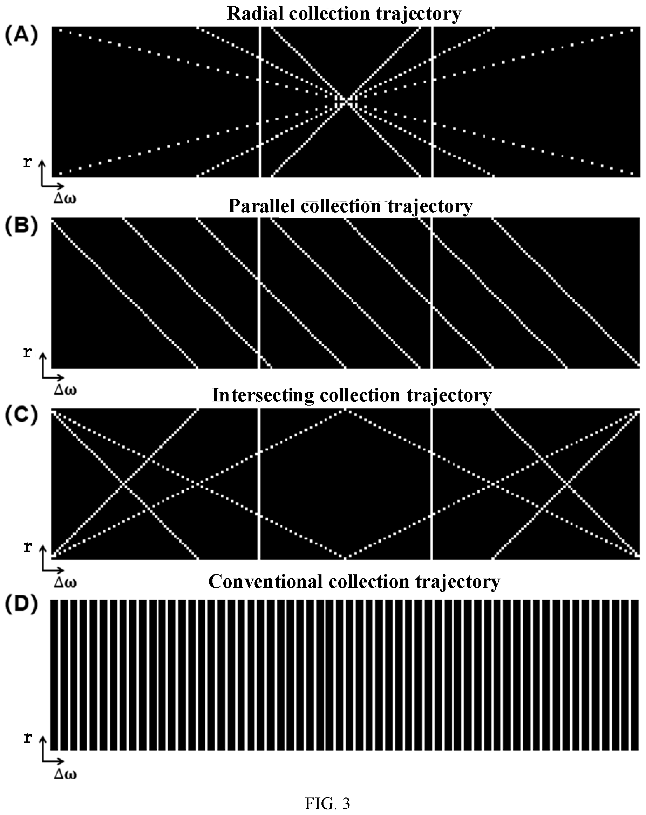

It should be noted that, in addition to being arranged in the radial pattern on the $r$-$\Delta\omega$ plane, the collected $n$ sets of CEST magnetic resonance raw images corresponding to the $n$ slices may also be arranged in a parallel pattern, an intersecting pattern, or the like, as illustrated in FIG. 3. A specific pattern can be determined based on the applied saturation pulse frequency and the variable saturation gradient.

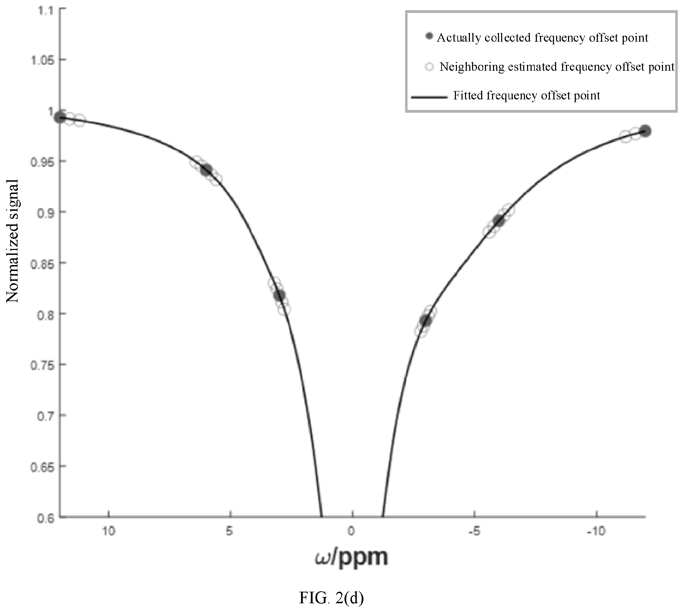

FIG. 2(d) illustrates that, in the collected raw images, a quantity of saturation frequency offset points actually collected for each slice in the $r$-direction is n, corresponding to $n$ saturation frequency offset points on the Z-spectrum. For the specific collection scheme illustrated in FIG. 2(a), the quantity of saturation frequency offset points actually collected for each slice in the $r$-direction is nine.

At S3, a target uncollected image is determined based on a collected image in the $r\text{-}\Delta\omega$ plane, and the target uncollected image is estimated.

The target uncollected image may include an uncollected image, in the $r\text{-}\Delta\omega$ plane, on an adjacent slice and at an adjacent saturation frequency offset relative to the collected image.

In some examples of the present disclosure, the collected images are denoted as $I_{r_1,\omega_1}$ and $I_{r_2,\omega_2}$, and the target uncollected images corresponding to the collected images include $I_{r_1,\omega_2}$ and $I_{r_2,\omega_1}$, and $I_{r_1,\omega_2}$ and $I_{r_2,\omega_1}$ are estimated through the following equations, respectively:

$$I_{r_1,\omega_2} = I_{r_1,\omega_1} \cdot \frac{\overline{I_{r_2,\omega_2}}}{\overline{I_{r_1,\omega_1}}} * \frac{\overline{M_{r_1}}}{\overline{M_{r_2}}}$$

$$I_{r_2,\omega_1} = I_{r_2,\omega_2} \cdot \frac{\overline{I_{r_1,\omega_1}}}{\overline{I_{r_2,\omega_2}}} * \frac{\overline{M_{r_2}}}{\overline{M_{r_1}}},$$

where $M_{r_1}$ and $M_{r_2}$ represent unsaturated images on slice $r1$ and slice $r2$, respectively; $I_{zr_1,\omega_1}$ and $I_{r_2,\omega_2}$ represent a collected image at frequency offset $\omega_1$ on the slice $r1$ and a collected image at frequency offset $\omega_2$ on the slice $r2$, respectively; $I_{r_1,\omega_2}$ and $I_{r_2,\omega_1}$ represent a target uncollected image at the frequency offset $\omega_2$ on the slice $r1$ and a target uncollected image at the frequency offset $\omega_1$ on the slice $r2$, respectively; $S_{r_1,\omega_2}$ and $S_{r_2,\omega_1}$ represent relative saturation information at the frequency offset $\omega_2$ on the slice $r1$ and relative saturation information at the frequency offset $\omega_1$ on the slice $r2$, respectively; $\overline{I_{r_2,\omega_2}}$ and $\overline{I_{r_1,\omega_1}}$ represent an average value of the collected image at the frequency offset $\omega_1$ on the slice $r1$ and an average value of the collected image at the frequency offset $\omega_2$ on the slice $r2$, respectively; and $\overline{M_{r_1}}$ and $\overline{M_{r_2}}$ represent average values of the unsaturated images on the slice $r1$ and the slice $r2$, respectively.

Specifically, the target uncollected image is estimated using a spatial-frequency offset nearest-neighbor estimation method. For two CEST MRI images on adjacent slices, if the two CEST MRI images have different saturation frequency offset information, the two CEST MRI images are expressed as:

$$I_{r_1,\omega_1} = M_{r_1} \cdot S_{r_1,\omega_1},$$

and

$$I_{r_2,\omega_2} = M_{r_2} \cdot S_{r_2,\omega_2},$$

where $\overline{M_{r1}}$ and $\overline{M_{r2}}$ can each be obtained through a single separate collection, and $S_{r_1,\omega_1}$ and $S_{r_2,\omega_2}$ represent the relative saturation information at the frequency offset $\omega_1$ on the slice $r1$ and the relative saturation information at the frequency offset $\omega_2$ on the slice $r2$, respectively, which cannot be obtained directly but can be calculated using equations

$$S_{r_1,\omega_1} = \frac{I_{r_1,\omega_1}}{M_{r_1}} \quad \text{and} \quad S_{r_2,\omega_2} = \frac{I_{r_2,\omega_2}}{M_{r_2}}.$$

Under ideal conditions, when the image $I_{r_1,\omega_2}$ at the saturation frequency offset $\omega_2$ on the slice $r1$ needs to be obtained, the image $I_{r_1,\omega_2}$ can be calculated using the following formula:

$$I_{r1,\omega_2} = I_{r1,\omega_1} \cdot \frac{S_{r1,\omega_2}}{S_{r1,\omega_1}}.$$

However, in practice, a single collection of a conventional CEST sequence can only obtain an image at one saturation frequency offset (such as $\omega_1$). That is, $S_{r_1,\omega_2}$ is unknown. With a gradient-edited sequence provided in the present disclosure, although only the raw image at the saturation frequency offset $\omega_1$ is collected on the slice $r1$, but the raw image at the saturation frequency offset $\omega_2$ is collected on the slice $r2$ adjacent to the slice $r1$. Due to a spatial-spectral high-resolution characteristic of the provided sequence, anatomical information and metabolic information on adjacent slices have relatively high similarity. That is, r1 is relatively close to r2, and $\omega_1$ is relatively close to $\omega_2$. Therefore, the actually uncollected image $I_{zr_1,\omega_2}$ can be estimated using the following formula:

$$I_{r_1, \omega_2} = M_{r_1} * S_{r_1, \omega_2} \approx I_{r_1, \omega_1} \cdot \frac{\overline{I_{r_2, \omega_2}}}{\overline{I_{r_1, \omega_1}}} * \frac{\overline{M_{r_1}}}{\overline{M_{r_2}}}.$$

Similarly, the uncollected raw image $I_{z2, \omega 1}$ for the slice $r2$ can also be approximately estimated:

$$I_{r_2, \omega_1} = M_{r_2} * S_{r_2, \omega_1} \approx I_{r_2, \omega_2} \cdot \frac{\overline{I_{r_1, \omega_1}}}{\overline{I_{r_2, \omega_2}}} * \frac{\overline{M_{r_2}}}{\overline{M_{r_1}}}.$$

At S4, interpolation and/or fitting is performed based on the collected image and the estimated target uncollected image to obtain a CEST or Z-spectrum image having a high spatial resolution and a high spectral sampling rate.

In some embodiments of the present disclosure, the performing the interpolation and/or the fitting based on the collected image and the estimated target uncollected image includes: performing simulation using a multi-pool Bloch-McConnell equation, and obtaining a Z-spectrum sample set with a plurality of different waveforms by varying a simulation parameter of each pool; performing singular value decomposition on the Z-spectrum sample set, and selecting right singular vectors corresponding to $m$ largest singular values to form a new singular vector matrix, where $m$ is an integer greater than 2; and determining all vectors z0 having incomplete saturation frequency offset points based on the collected image and the estimated target uncollected image, and obtaining, for each of all the vectors z0, a complete spectrum of a pixel point corresponding to the vector z0 based on the vector z0 and the new singular vector matrix.

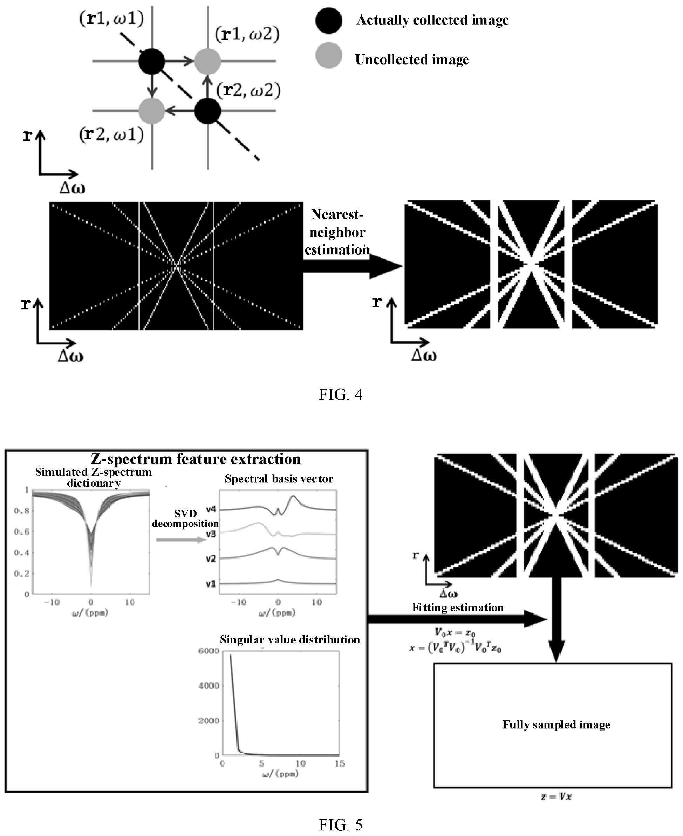

Specifically, a fitting estimation employs a partial separable model, as illustrated in FIG. 5.

As illustrated in FIG. 5, the simulation is performed using the multi-pool Bloch-McConnell equation. A large number of Z-spectra with different waveforms are obtained through varying the simulation parameter of each pool. With each Z-spectrum as a column, a two-dimensional matrix is formed, i.e., the Z-spectrum sample set D. Then, the Singular Value Decomposition (SVD) is performed on the Z-spectrum sample set:

$$D = U \cdot \Sigma \cdot V^{T},$$

where U, $\Sigma$, and V are all two-dimensional matrices. Each row in U represents a combination coefficient of each Z-spectrum in the sample set D with respect to a basis matrix V. Each column in V represents a different singular vector, i.e., a basis capable of characterizing a shape of the Z-spectrum. $\Sigma$ represents a diagonal matrix. Elements on a diagonal are singular values corresponding to right singular vectors in V, arranged in a descending order.

The right singular vectors corresponding to the $m$ (e.g., three to five) largest singular values are selected based on magnitudes of the singular values in $\Sigma$ to form the new singular vector matrix V. In this case, for the one-dimensional vector z0 having incomplete saturation frequency offset points, a coefficient x of each vector in V can be fitted using the following formula:

$$x = \left(V_0{}^T V_0\right)^{-1} V_0{}^T z_0,$$

where $V_0$ represents data of a principal component V of Z-spectrum information at existing saturation frequency offset points.

As illustrated in FIG. 5, through the coefficient x obtained through fitting, complete high-resolution spectrum information can be estimated:

$$z = Vx,$$

where z represents a complete spectrum obtained after an estimation for a predetermined pixel point. By repeating the above fitting process for all pixel points in a three-dimensional space, a whole-brain spatial-spectral high-resolution CEST MRI image, i.e., a fully sampled image in FIG. 5, can be obtained.

In some embodiments of the present disclosure, the variable saturation gradient-based three-dimensional CEST or Z-spectrum magnetic resonance imaging method further includes performing a post-processing on the obtained CEST or Z-spectrum image having the high spatial resolution and the high spectral sampling rate. The post-processing includes a magnetization transfer ratio asymmetry processing, Lorentzian difference, and a multi-pool Lorentzian processing.

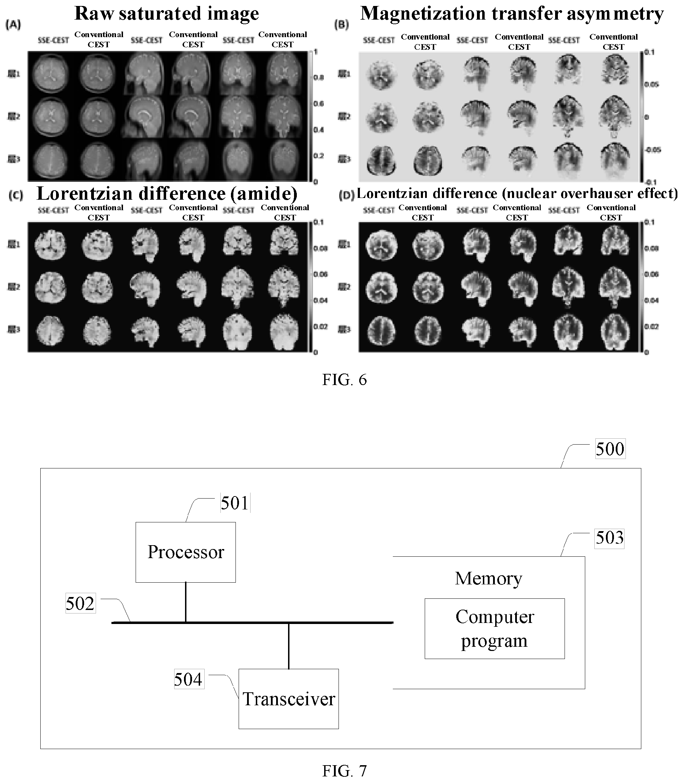

Specifically, as illustrated in FIG. 6, taking the whole brain as an example, the post-processing is performed on a reconstructed whole-brain high-resolution CEST MRI image to obtain a CEST-weighted contrast image. The post-processing includes Magnetization Transfer Ratio (MTR) asymmetry, Lorentzian difference, and other processings. As illustrated in FIG. 6, compared with a conventional CEST image obtained using conventional techniques (using independent collection and interpolation techniques), an SSE CEST image obtained using the method of the present disclosure (the post-processing after *n* collections) has comparable resolution, spectral sampling rate, etc. That is, the present disclosure can obtain the CEST image having the high spatial resolution and the high spectral sampling rate through a small number of collections.

In summary, the variable saturation gradient-based three-dimensional CEST or Z-spectrum magnetic resonance imaging method according to the embodiments of the present disclosure has the following advantages and advantageous effects.

First, a fast collection speed is realized. By designing a variable saturation gradient-based r-w collection trajectory, spatial encoding in the *r*-direction and saturation frequency offset encoding are performed and can be effectively separated. This method avoids a need for separate saturation labeling and collection for each frequency offset. In comparison with a conventional frequency-offset-by-frequency-offset CEST collection sequence, more than thirty-fold collection acceleration can be achieved under a same frequency offset sampling rate.

Second, both the high spatial resolution and the high spectral sampling rate are achieved. The solution provided in the present disclosure utilizes redundant information and prior knowledge in both spatial and spectral dimensions. By further combining the variable saturation gradient-based r-w collection trajectory with a CEST Z-spectrum lineshape simulation model, a whole-organ metabolic scan, such as a whole-brain metabolic scan and a whole-liver metabolic scan, having the high spatial resolution and the high Z-spectrum sampling rate can be reconstructed, which revolutionarily expands application scenarios and improves accuracy of CEST, and achieves reduced sensitivity to movements and field inhomogeneity.

Third, high flexibility is realized. A variable saturation gradient collection sequence included in the present disclosure allows for design and optimization of different r-w collection trajectories, such as radial, parallel, and intersecting trajectories, tailored to different application scenarios and target metabolites to be detected, achieving CEST magnetic resonance imaging and analysis of various metabolic components in the human body, including proteins, lipids, glucose, creatine, salicylic acid, and the like.

Fourth, strong scalability is obtained. The method provided in the present disclosure is not limited to being applied to CEST brain scanning at 3 T magnetic resonance. Further, the method provided in the present disclosure can also be applied to parts throughout the body, including but not limited to, abdominal organs, thoracic organs, muscles, etc., and magnetic resonance imaging devices with different field strengths (1.5 T, 3 T, 5 T, 7 T, 9.4 T, and 11.7 T). The collection sequence based on variable saturation gradient encoding can serve as an independent CEST labeling module, and be combined with an existing multi-channel parallel collection, compressed sensing-based, and deep learning-based water signal readout and reconstruction module to further improve a collection speed, making the method especially suitable for CEST metabolic imaging of moving organs such as the heart and the liver.

FIG. 7 is a structural block diagram of an electronic device according to an embodiment of the present disclosure.

As illustrated in FIG. 7, an electronic device 500 includes a processor 501 and a memory 503. The processor 501 and the memory 503 are connected via, e.g., a bus 502. Alternatively, the electronic device 500 may further include a transceiver 504. It should be noted that, in practical applications, one or more transceivers 504 may be provided. A structure of the electronic device 500 does not constitute a limitation to the embodiments of the present disclosure.

The processor 501 may be a Central Processing Unit (CPU), a general-purpose processor, a Digital Signal Processor (DSP), an Application Specific Integrated Circuit (ASIC), a Field Programmable Gate Array (FPGA) or other programmable logic devices, a transistor logic device, a hardware component, or any combination thereof. Various exemplary logical blocks, modules, and circuits described in conjunction with the content disclosed herein may be implemented or performed. The processor 501 may also be a combination implementing computing functions, such as a combination including one or more microprocessors and a combination of a DSP and a microprocessor.

The bus 502 may include a path for transmitting information between the above components. The bus 502 may be a Peripheral Component Interconnect (PCI) bus, an Extended Industry Standard Architecture (EISA) bus, or the like. The buses 502 may be divided into an address bus, a data bus, a control bus, etc. For the convenience of description, only one thick line is used in FIG. 7, but it does not mean that there is only one bus or one type of bus.

The memory 503 is configured to store a computer program corresponding to the variable saturation gradient-based three-dimensional CEST magnetic resonance imaging method according to the above embodiments of the present disclosure. The computer program is controlled and executed by the processor 501. The processor 501 is configured to execute the computer program stored in the memory 503 to implement the content shown in the above method embodiments.

The electronic device 500 may include, but is not limited to, a mobile terminal such as a mobile phone, a laptop computer, a digital broadcast receiver, a Personal Digital Assistant (PDA), a PAD, a Portable Multimedia Player (PMP), and a vehicle-mounted terminal (e.g., a vehicle-mounted navigation terminal), and a fixed terminal such as a digital TV and a desktop computer. The electronic device 500 illustrated in FIG. 7 is exemplary only, and should not be construed as limiting the function and scope of use of the embodiments of the present disclosure.

It should be noted that, the logics and/or steps represented in the flowchart or described otherwise herein can be for example considered as a list of ordered executable instructions for implementing logic functions, and can be embodied in any computer-readable medium that is to be used by or used with an instruction execution system, apparatus, or device (such as a computer-based system, a system including a processor, or any other system that can retrieve and execute instructions from an instruction execution system, apparatus, or device). For the present disclosure, a "computer-readable medium" can be any apparatus that can contain, store, communicate, propagate, or transmit a program to be used by or used with an instruction execution system, apparatus, or device. More specific examples of computer-readable mediums include, as a non-exhaustive list: an electrical connector (electronic device) with one or more wirings, a portable computer disk case (magnetic devices), a Random Access Memory (RAM), a Read Only Memory (ROM), an Erasable Programmable Read Only Memory (EPROM or flash memory), a fiber optic device, and a portable Compact Disk Read Only memory (CDROM). In addition, the computer-readable medium may even be paper or other suitable medium on which the program can be printed, as the program can be obtained electronically, e.g., by optically scanning the paper or the other medium, and then editing, interpreting, or otherwise processing the scanning result when necessary, and then stored in a computer memory.

It should be understood that each part of the present disclosure may be realized by hardware, software, firmware, or a combination thereof. In the above embodiments, a plurality of steps or methods may be realized by software or firmware stored in the memory and executed by an appropriate instruction execution system. For example, when it is realized by the hardware, likewise in another embodiment, the steps or methods may be realized by one or a combination of the following techniques known in the art: a discrete logic circuit having a logic gate circuit for realizing a logic function of a data signal, an application-specific integrated circuit having an appropriate combination logic gate circuit, a Programmable Gate Array (PGA), a Field Programmable Gate Array (FPGA), etc.

Reference throughout this specification to "an embodiment", "some embodiments", "an example", "a specific example", or "some examples" means that a particular feature, structure, material, or characteristic described in connection with the embodiment or example is included in at least one embodiment or example of the present disclosure. The appearances of the above phrases in various places throughout this specification are not necessarily referring to the same embodiment or example. Further, the particular features, structures, materials, or characteristics may be combined in any suitable manner in one or more embodiments or examples.

In the description of the present disclosure, the orientation or the position indicated by terms such as "center", "longitudinal", "transverse", "length", "width", "thickness", "over", "below", "front", "rear", "left", "right", "vertical", "horizontal", "top", "bottom", "inner", "outer", "clockwise", "anti-clockwise", "axial", "radial", and "circumferential" should be construed to refer to the orientation or the position as shown in the drawings, and is only for the convenience of describing the embodiments of the present disclosure and simplifying the description, rather than indicating or implying that the pointed device or element must have a specific orientation, or be constructed and operated in a specific orientation, and therefore cannot be understood as a limitation of the present disclosure.

In addition, the terms "first" and "second" are only used for descriptive purposes, and cannot be understood as indicating or implying relative importance or implicitly indicating the number of indicated technical features. Therefore, the features associated with "first" and "second" may explicitly or implicitly include at least one of the features or more of the features. In the description of the present disclosure, "plurality" means at least two, unless otherwise specifically defined.

In the present disclosure, unless otherwise clearly specified and limited, terms such as "install", "connect", "connect to", and "fix" should be understood in a broad sense. For example, it may be a fixed connection or a detachable connection or connection as one piece; mechanical connection or electrical connection; direct connection or indirect connection through an intermediate; internal communication of two components or the interaction relationship between two components, unless otherwise clearly limited. For those of ordinary skill in the art, the specific meaning of the above-mentioned terms in the present disclosure can be understood according to specific circumstances.

In the present disclosure, unless expressly stipulated and defined otherwise, the first feature "on" or "under" the second feature may mean that the first feature is in direct contact with the second feature, or the first and second features are in indirect contact through an intermediate. Moreover, the first feature "above" the second feature may mean that the first feature is directly above or obliquely above the second feature, or simply mean that the level of the first feature is higher than that of the second feature. The first feature "below" the second feature may mean that the first feature is directly below or obliquely below the second feature, or simply mean that the level of the first feature is smaller than that of the second feature.

Although embodiments of the present disclosure have been shown and described above, it should be understood that the above embodiments are merely exemplary, and cannot be construed as limiting the present disclosure. For those skilled in the art, changes, alternatives, and modifications can be made to the embodiments without departing from the scope of the present disclosure.

**Claims**

1. A variable saturation gradient-based three-dimensional Chemical Exchange Saturation Transfer, CEST, or Z-spectrum magnetic resonance imaging method, comprising:

   applying a saturation pulse to a target object while turning on a gradient in an *r*-direction, reading out a set of raw images, and repeating the above process *n* times to obtain *n* sets of raw images, wherein a center frequency of the saturation pulse applied and a value of the gradient are different for each time, wherein *r* represents a spatial position and comprises at least one of *x*-direction, *y*-direction, or *z*-direction, and wherein *n* is an integer greater than 1;
   filling the *n* sets of raw images into an *r*-$\Delta\omega$ plane, where $\Delta\omega$ represents a frequency offset;
   determining a target uncollected image based on a collected image in the *r*-$\Delta\omega$ plane, and estimating the target uncollected image; and
   performing interpolation and/or fitting based on the collected image and the estimated target uncollected image to obtain a CEST or Z-spectrum image having a high spatial resolution and a high spectral sampling rate.

2. The variable saturation gradient-based three-dimensional CEST or Z-spectrum magnetic resonance imaging method according to claim 1, wherein the *n* sets of raw images are filled into the *r*-$\Delta\omega$ plane using the following equation:

$$\Delta\omega(r) = \Delta\omega_0 + \gamma G_{\text{offset}} \Delta r \, ,$$

   where $\Delta\omega(r)$ represents a frequency offset corresponding to the *r*-direction, $\Delta r$ represents a distance between an imaging position corresponding to the *r*-direction and a magnetic field zero point, $\gamma$ represents a gyromagnetic ratio, $\Delta\omega_0$ represents a frequency offset between a saturation pulse frequency and a water proton, and $G_{\text{offset}}$ represents the gradient in the *r*-direction that is turned on during application of the saturation pulse.

3. The variable saturation gradient-based three-dimensional CEST or Z-spectrum magnetic resonance imaging method according to claim 1 or 2, wherein the target uncollected image comprises an uncollected image, in the *r*-$\Delta\omega$ plane, on an adjacent slice and at an adjacent saturation frequency offset relative to the collected image.

4. The variable saturation gradient-based three-dimensional CEST or Z-spectrum magnetic resonance imaging method according to any one of claims 1 to 3, wherein the collected images are denoted as $I_{r_1, \omega_1}$ and $I_{r_1, \omega_2}$, and wherein the target uncollected images corresponding to the collected images comprise $I_{r_1, \omega_2}$ and $I_{r_2, \omega_1}$, and $I_{r_1, \omega_2}$ and $I_{r_2, \omega_1}$ are estimated through the following equations, respectively:

$$I_{r_1, \omega_2} = I_{r_1, \omega_1} \cdot \frac{\overline{I_{r_2, \omega_2}}}{\overline{I_{r_1, \omega_1}}} * \frac{\overline{M_{r_1}}}{\overline{M_{r_2}}}$$

$$I_{r_2, \omega_1} = I_{r_2, \omega_2} \cdot \frac{\overline{I_{r_1, \omega_1}}}{\overline{I_{r_2, \omega_2}}} * \frac{\overline{M_{r_2}}}{\overline{M_{r_1}}} \, ,$$

   where $M_{r_1}$ and $M_{r_2}$ represent unsaturated images on slice *r*1 and slice *r*2, respectively; $I_{zr_1, \omega_1}$ and $I_{zr_2, \omega_2}$ represent a collected image at frequency offset $\omega_1$ on the slice *r*1 and a collected image at frequency offset $\omega_2$ on the slice *r*2, respectively; $I_{r_1, \omega_2}$ and $I_{r_1, \omega_1}$ represent a target uncollected image at the frequency offset $\omega_2$ on the slice *r*1 and a target uncollected image at the frequency offset $\omega_1$ on the slice *r*2, respectively; $S_{r_1, \omega_2}$ and $S_{r_1, \omega_2}$ represent relative saturation information at the frequency offset $\omega_2$ on the slice *r*1 and relative saturation information at the frequency offset $\omega_1$ on the slice *r*2, respectively; $\overline{I_{r_2, \omega_2}}$ and $\overline{I_{r_1, \omega_1}}$ represent an average value of the collected image at the frequency offset $\omega_1$ on the slice *r*1 and an average value of the collected image at the frequency offset $\omega_2$ on the slice *r*2, respectively; and $\overline{M_{r_1}}$ and $\overline{M_{r_2}}$ represent average values of the unsaturated images on the slice *r*1 and the slice *r*2, respectively.

5. The variable saturation gradient-based three-dimensional CEST or Z-spectrum magnetic resonance imaging method according to any one of claims 1 to 4, wherein said performing the interpolation and/or the fitting based on the collected image and the estimated target uncollected image comprises:

performing simulation using a multi-pool Bloch-McConnell equation, and obtaining a Z-spectrum sample set with a plurality of different waveforms by varying a simulation parameter of each pool;

performing singular value decomposition on the Z-spectrum sample set, and selecting right singular vectors corresponding to $m$ largest singular values to form a new singular vector matrix, where $m$ is an integer greater than 2; and

determining all vectors z0 having incomplete saturation frequency offset points based on the collected image and the estimated target uncollected image, and obtaining, for each of all the vectors z0, a complete spectrum of a pixel point corresponding to the vector z0 based on the vector z0 and the new singular vector matrix.

6. The variable saturation gradient-based three-dimensional CEST or Z-spectrum magnetic resonance imaging method according to any one of claims 1 to 5, wherein a value of the gradient comprises at least 0.

7. The variable saturation gradient-based three-dimensional CEST or Z-spectrum magnetic resonance imaging method according to any one of claims 1 to 6, wherein the $n$ sets of raw images corresponding to $n$ slices are collected using a fast spin echo method, a gradient echo method, or an echo planar method.

8. The variable saturation gradient-based three-dimensional CEST or Z-spectrum magnetic resonance imaging method according to any one of claims 1 to 7, wherein the $n$ sets of raw images collected corresponding to $n$ slices are arranged in a radial pattern, a parallel pattern, or an intersecting pattern on the $r$-$\Delta\omega$ plane.

9. The variable saturation gradient-based three-dimensional CEST or Z-spectrum magnetic resonance imaging method according to any one of claims 1 to 8, further comprising:

performing a post-processing on the obtained CEST or Z-spectrum image having the high spatial resolution and the high spectral sampling rate, wherein the post-processing comprises a magnetization transfer ratio asymmetry processing, Lorentzian difference, and a multi-pool Lorentzian processing.

10. An electronic device, comprising:

a memory;
a processor; and
a computer program stored on the memory, wherein the computer program, when executed by the processor, implements the variable saturation gradient-based three-dimensional CEST or Z-spectrum magnetic resonance imaging method according to any one of claims 1 to 9.

Applying a saturation pulse to a target object while turning on a gradient in an $r$-direction, reading out a raw image, and repeating the above process $n$ times to obtain $n$ sets of raw images, in which a center frequency of the saturation pulse applied each time and a value of the gradient are different, $r$ represents a spatial position and includes at least one of $x$-direction, $y$-direction, or $z$-direction, and $n$ is an integer greater than 1 — S1

Filling the $n$ sets of raw images into an $r$-$\Delta\omega$ plane, where $\Delta\omega$ represents a frequency offset — S2

Determining a target uncollected image based on a collected image in the $r$-$\Delta\omega$ plane, and estimating the target uncollected image — S3

Performing interpolation and/or fitting based on the collected image and the estimated target uncollected image to obtain a CEST or Z-spectrum image having a high spatial resolution and a high spectral sampling rate — S4

FIG. 1

FIG. 2(a)

FIG. 2(b)

FIG. 2(c)

FIG. 2(d)

**Radial collection trajectory**

**(A)**

r

$\Delta\omega$

**Parallel collection trajectory**

**(B)**

r

$\Delta\omega$

**Intersecting collection trajectory**

**(C)**

r

$\Delta\omega$

**Conventional collection trajectory**

**(D)**

r

$\Delta\omega$

FIG. 3

FIG. 4

FIG. 5

**Raw saturated image**

(A)

**Magnetization transfer asymmetry**

(B)

(C) **Lorentzian difference (amide)**

(D) **Lorentzian difference (nuclear overhauser effect)**

FIG. 6

FIG. 7

## INTERNATIONAL SEARCH REPORT

| | International application No. |
|---|---|
| | **PCT/CN2023/125056** |

**A. CLASSIFICATION OF SUBJECT MATTER**

A61B5/055(2006.01)i

According to International Patent Classification (IPC) or to both national classification and IPC

**B. FIELDS SEARCHED**

Minimum documentation searched (classification system followed by classification symbols)

IPC:A61B 5/-

Documentation searched other than minimum documentation to the extent that such documents are included in the fields searched

Electronic data base consulted during the international search (name of data base and, where practicable, search terms used)

CNTXT, ENTXT, WPABS: "cest", 化学, 交换, 饱和, 转移, 估计, 插值, r方向, 径向, 磁共振, 梯度, 可变, k空间, RF, 射频, magnetic, resonance, imaging, sequence, magnetization, transfer, chemical, exchange, saturation, radio, frequency

**C. DOCUMENTS CONSIDERED TO BE RELEVANT**

| Category* | Citation of document, with indication, where appropriate, of the relevant passages | Relevant to claim No. |
|---|---|---|
| A | US 2018299525 A1 (TOSHIBA MEDICAL SYSTEMS CORP.) 18 October 2018 (2018-10-18) description, paragraphs [0037]-[0040], and figures 1-2 | 1-10 |
| A | CN 105759233 A (SHENZHEN INSTITUTE OF ADVANCED TECHNOLOGY) 13 July 2016 (2016-07-13) entire document | 1-10 |
| A | CN 111722167 A (ZHEJIANG UNIVERSITY et al.) 29 September 2020 (2020-09-29) entire document | 1-10 |
| A | CN 114216920 A (TSINGHUA UNIVERSITY) 22 March 2022 (2022-03-22) entire document | 1-10 |
| A | US 2010286502 A1 (THE JOHNS HOPKINS UNIVERSITY et al.) 11 November 2010 (2010-11-11) entire document | 1-10 |
| A | US 2016334485 A1 (GENERAL ELECTRIC CO.) 17 November 2016 (2016-11-17) entire document | 1-10 |

☐ Further documents are listed in the continuation of Box C.    ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **04 December 2023** | **11 December 2023** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**
**Information on patent family members**

International application No.

**PCT/CN2023/125056**

| Patent document cited in search report | | | Publication date (day/month/year) | Patent family member(s) | | | Publication date (day/month/year) |
|---|---|---|---|---|---|---|---|
| US | 2018299525 | A1 | 18 October 2018 | US | 10481232 | B2 | 19 November 2019 |
| | | | | JP | 2018175829 | A | 15 November 2018 |
| | | | | JP | 7166747 | B2 | 08 November 2022 |
| CN | 105759233 | A | 13 July 2016 | CN | 105759233 | B | 25 September 2018 |
| CN | 111722167 | A | 29 September 2020 | US | 2020300949 | A1 | 24 September 2020 |
| | | | | CN | 111722167 | B | 30 July 2021 |
| CN | 114216920 | A | 22 March 2022 | None | | | |
| US | 2010286502 | A1 | 11 November 2010 | EP | 2205986 | A1 | 14 July 2010 |
| | | | | EP | 2205986 | B1 | 03 July 2013 |
| | | | | US | 8536866 | B2 | 17 September 2013 |
| | | | | WO | 2009042881 | A1 | 02 April 2009 |
| US | 2016334485 | A1 | 17 November 2016 | JP | 2016214301 | A | 22 December 2016 |
| | | | | JP | 6348449 | B2 | 27 June 2018 |
| | | | | US | 10234527 | B2 | 19 March 2019 |

Form PCT/ISA/210 (patent family annex) (July 2022)

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202311266353